Europäisches Patentamt

European Patent Office

Office européen des brevets

(19)

(11) Numéro de publication: **0 395 458**
**A1**

## (12) DEMANDE DE BREVET EUROPEEN

(21) Numéro de dépôt: 90400756.4

(22) Date de dépôt: **20.03.90**

(51) Int. Cl.⁵: **G03B 42/02, A61B 6/00**

(30) Priorité: 28.04.89 FR 8905666

(43) Date de publication de la demande:
**31.10.90 Bulletin 90/44**

(84) Etats contractants désignés:
**DE ES GB IT NL**

(71) Demandeur: **GENERAL ELECTRIC CGR S.A.**
**100, rue Camille-Desmoulins**
**F-92130 Issy les Moulineaux(FR)**

(72) Inventeur: **Tirelli, Marco**

Cabinet Ballot-Schmit, 7, rue Le Sueur
**F-75116 Paris(FR)**
Inventeur: **Rouchy, Didier**
Cabinet Ballot-Schmit, 7, rue Le Sueur
**F-75116 Paris(FR)**
Inventeur: **Rapeau, Jean-Claude**
Cabinet Ballot-Schmit, 7, rue Le Sueur
**F-75116 Paris(FR)**

(74) Mandataire: **Ballot, Paul Denis Jacques et al**
**Cabinet Ballot-Schmit 7, rue le Sueur**
**F-75116 Paris(FR)**

(54) **Porte-cassette adaptable en dimension et en position pour mammographie.**

(57) L'invention concerne les mammographes et plus particulièrement un dispositif qui permet son adaptation aux différentes tailles de cassette et le déplacement de cette dernière.

Le dispositif comprend essentiellement un curseur 20 qui, en se déplaçant, adapte l'écartement des ergots 23 et 24 de maintien aux nouvelles dimensions latérales de la cassette 18 tout en venant presser la cassette 18 contre un bord de référence 51 de la table 8. Par ailleurs, le curseur 20 est porté par une platine 21 qui se déplace de manière que l'un ou l'autre des côtés latéraux de la cassette (18) s'aligne sur le côté latéral correspondant de la table 8.

L'invention est applicable aux mammographes.

FIG. 2

EP 0 395 458 A1

# PORTE-CASSETTE ADAPTABLE EN DIMENSION ET EN POSITION POUR MAMMOGRAPHIE

L'invention concerne les appareils de mammographie et, plus particulièrement dans de tels appareils, les dispositifs de support de la cassette contenant le film sensible.

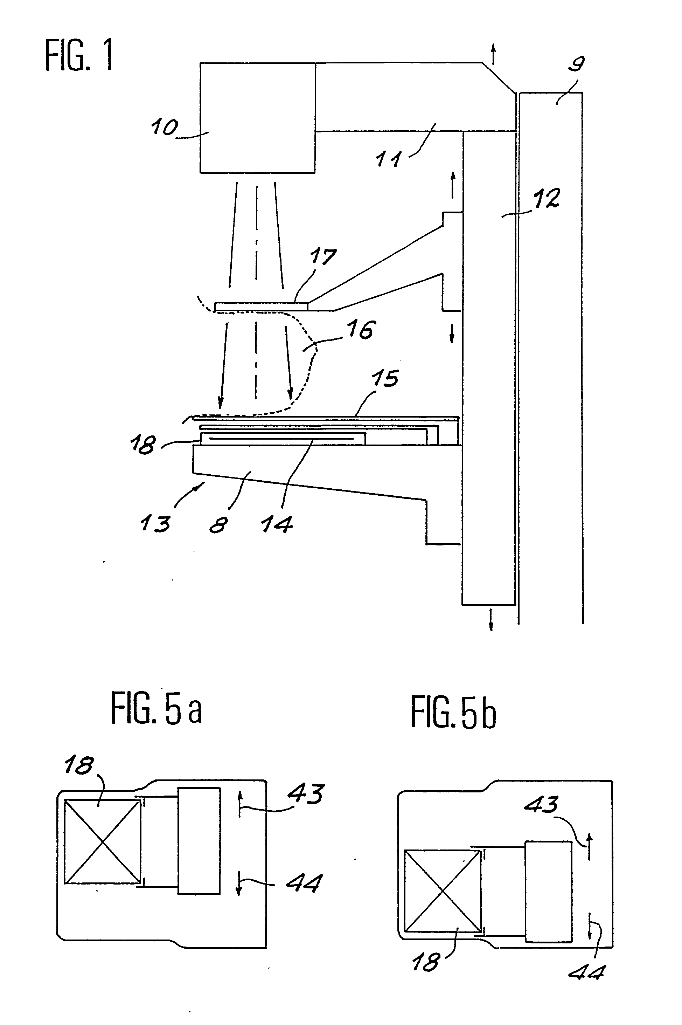

Les systèmes de radiologie du type mammographe comprennent, comme le montre la figure 1, une source 10 de rayonnement X portée par une potence 11 disposée au sommet d'une plaque verticale 12. Cette dernière comporte un ensemble 13 sur lequel repose le sein 16 à examiner par l'intermédiaire d'une tablette horizontale 15. Une pelote 17, transparente au rayonnement X et mobile verticalement sur la plaque 12, sert à comprimer le sein.

Pour s'adapter à la taille de la patiente, la plaque 12 est montée sur une colonne verticale 9 reposant sur le sol et se déplace verticalement sur ladite colonne grâce à un dispositif mécanique approprié.

L'ensemble 13 présente, sur sa partie supérieure et sous la tablette 15, un tunnel dans lequel vient se loger un dispositif destiné à accepter une cassette 18. Cette cassette est constituée d'une boîte noire renfermant au moins un film 14 sensible au rayonnement X direct ou à un rayonnement photonique émis par un écran (non représenté) recevant le rayonnement X. C'est sur le film que se forme l'image latente du sein après une durée de pose appropriée; le développement du film donne un cliché radiographique.

Le dispositif contenant la cassette, appelé support de cassette, est un élément amovible que l'on glisse dans le tunnel avant la pose et dans lequel on introduit la cassette. Les films sensibles ont des dimensions normalisées, par exemple 18 cm x 24 cm ou 24 cm x 30 cm, et il en est de même des cassettes qui sont prévues pour les contenir ainsi que des supports de cassette.

Pour passer d'une cassette de dimensions données à une autre ayant des dimensions différentes, il faut habituellement changer en même temps le support de cassette. Il est donc en général prévu un support de cassette adapté aux dimensions de la cassette et qui constitue en quelque sorte un dispositif adaptateur qui est associé à chaque type de cassette. Il en résulte alors un coût supplémentaire lorsqu'on souhaite utiliser un mammographe pour toutes les dimensions habituelles des cassettes ainsi que la manipulation d'éléments encombrants tels que le support de cassette pendant l'examen. Ces inconvénients sont encore plus prononcés lorsque le support de cassette comporte une grille antidiffusante mobile 19, disposée entre la tablette 15 et la cassette 18, qui en augmente le coût et le poids. Un but de la présente invention est donc de réaliser un support de cassette qui s'adapte à toutes les dimensions de cassettes qui sont utilisées dans ce domaine.

L'avantage apporté par la présente invention est encore plus important lorsque le support de cassette comporte en outre une grille antidiffusante mobile. En effet, dans ce cas, le support de cassette inclut le mécanisme de déplacement de la grille antidiffusante, ce qui augmente le coût.

Aussi, un autre but de la présente invention est donc de réaliser un support de cassette qui conduit à n'utiliser qu'un seul mécanisme de déplacement de la grille antidiffusante.

La tablette horizontale 15 a, pour un mammographe donné, des dimensions extérieures adaptées aux dimensions de la cassette la plus grande qu'il est prévu d'utiliser. Lorsque l'on passe à des dimensions plus petites de cassette, cette dernière doit être positionnée à l'intérieur des dimensions plus grandes de la tablette 15 de manière que les côtés latéraux des cassettes qui sont proches du bord extérieur de la tablette 15 (côté de référence) soient alignés avec celui-ci. Il faut donc que le support de cassette qui est utilisé soit adapté à la position recherchée, ce qui conduit à avoir autant de supports de cassette que de cassettes différentes.

Un autre but de la présente invention est donc de réaliser un dispositif porte-cassette qui permet de changer la position de la cassette par rapport à celle de la tablette tout en conservant le côté de référence. L'invention se rapporte à un dispositif porte-cassette pour supporter sur une table une cassette contenant au moins un film sensible, caractérisé en ce qu'il comprend :

- une platine qui est montée mobile par rapport à la table suivant une première direction grâce à un premier dispositif de déplacement,

- un curseur qui est monté mobile par rapport à la platine suivant une deuxième direction perpendiculaire à la première grâce à un deuxième dispositif de déplacement, et

- des moyens associés au curseur pour maintenir la cassette en position par rapport à la platine et s'adapter aux dimensions de la cassette.

Le premier dispositif de déplacement comprend au moins deux guides sur lesquels coulissent des pattes solidaires de la platine, l'un des guides étant fileté et coopérant avec une noix d'entraînement de manière que la rotation du guide fileté déplace la platine dans le sens souhaité tandis que le second guide est lisse.

Le deuxième dispositif de déplacement comprend au moins un guide qui est solidaire de la platine et disposé perpendiculairement aux guides du premier dispositif de déplacement et sur lequel coulisse le curseur de manière à prendre deux positions correspondant chacune à une gorge de verrouillage qui est portée par ledit guide et qui coopère avec des moyens de verrouillage/déverrouillage portés par le curseur.

Les moyens de maintien de la cassette associés au curseur comprennent deux ergots qui sont portés par des bras portés par le curseur et qui viennent saisir deux des quatre coins de la cassette, lesdits bras comportent chacun un taquet qui coopère avec une première ouverture pratiquée dans le curseur et avec une deuxième ouverture pratiquée dans la platine de sorte que le déplacement du curseur entraîne la modification de l'écartement des ergots pour s'adapter aux dimensions de la cassette.

D'autres caractéristiques et avantages de la présente invention apparaîtront à la lecture de la description suivante d'un exemple particulier de réalisation, ladite description étant faite en relation avec les dessins joints dans lesquels :

- la figure 1 est une vue en coupe schématique d'un mammographe,
- la figure 2 est une vue de dessus du dispositif porte-cassette selon l'invention, abstraction faite de la grille antidiffusante et de la tablette,
- la figure 3 est une vue en coupe schématique suivant un plan vertical du dispositif porte-cassette selon l'invention,

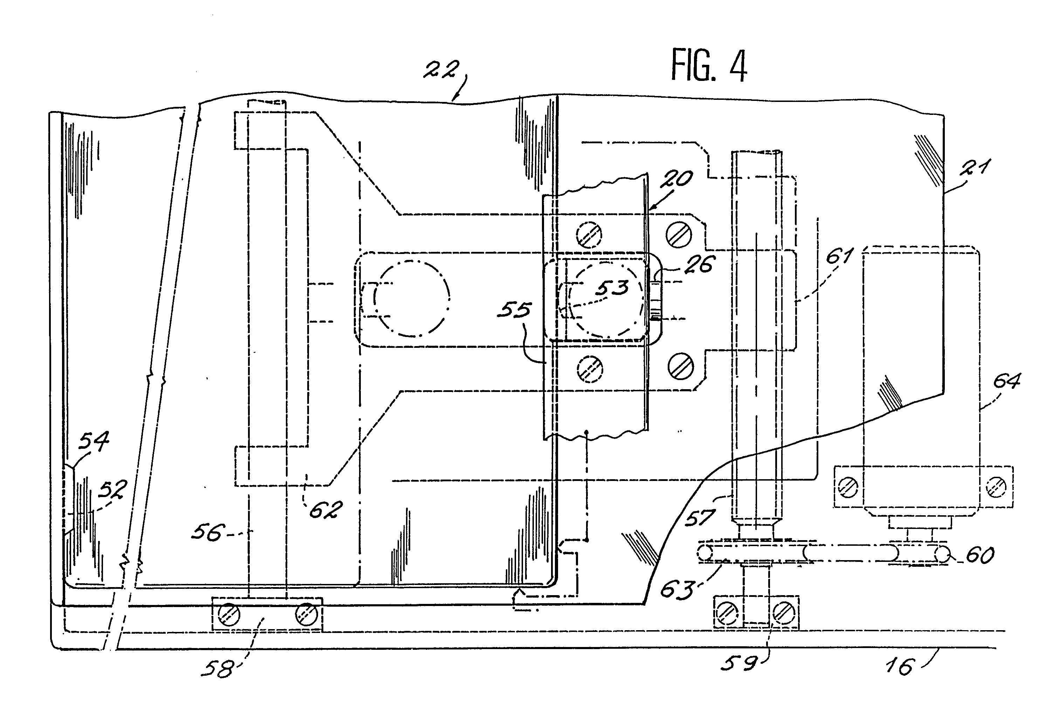

- la figure 4 est une vue de dessus montrant le mécanisme de déplacement latéral de la cassette,
- les figures 5-a et 5-b illustrent deux positions de la cassette qui peuvent être obtenues par le dispositif porte-cassette selon l'invention, et
- la figure 6 est une vue explicative partielle suivant la flèche F de la figure 3.

L'invention sera décrite dans son application à un dispositif porte-cassette qui s'adapte à deux tailles de cassettes, par exemple la taille 24 cm x 30 cm et celle de 18 cm x 24 cm.

Sur les figures, la référence 8 indique la table sur laquelle repose le dispositif porte-cassette selon l'invention. Ce dernier comprend essentiellement un curseur 20 qui porte des ergots 23 et 24 de maintien d'une cassette 18, une platine 21 qui supporte le curseur 20 et un mécanisme de déplacement 22 de la platine 21. Le curseur 20 est constitué d'une pièce 25 qui coulisse sur un guide 26 suivant les directions indiquées par les flèches 27 et 28. Le guide 26 est fixé à la platine 21 par l'intermédiaire de pattes 29 et 30 et de vis 31 et 32 pour la fixation des pattes sur la platine et d'une vis 33 pour la fixation du guide dans les pattes. Le guide 26 comporte deux gorges 34 et 35 qui correspondent chacune à une position du curseur 20 sur le guide 26. Ces gorges coopèrent avec un cran de verrouillage 36 porté par le curseur 20, ce cran étant maintenu dans la gorge 34 ou 35 par un ressort 37. Un bouton 38 sert à déverrouiller le cran et à déplacer le curseur d'une gorge à l'autre.

Le curseur 20 comporte deux extrémités latérales 39 et 40 qui portent chacune respectivement un bras 41 et 42 de maintien de la cassette 18 à deux coins de celle-ci par l'intermédiaire des ergots 23 et 24. L'extrémité de chaque bras 41 ou 42, opposée à celle de l'ergot, porte un taquet 45 ou 46. Le taquet 45 coopère avec deux ouvertures, l'une 47 pratiquée dans le curseur parallèlement au bord 51 de la cassette 18 et l'autre 49 pratiquée dans la platine suivant une direction oblique par rapport au bord 51. De même, le taquet 46 coopère avec deux ouvertures, l'une 48 pratiquée dans le curseur parallèlement au bord 51 de la cassette 18 et l'autre 50 pratiquée dans la platine suivant une direction oblique par rapport audit bord 51.

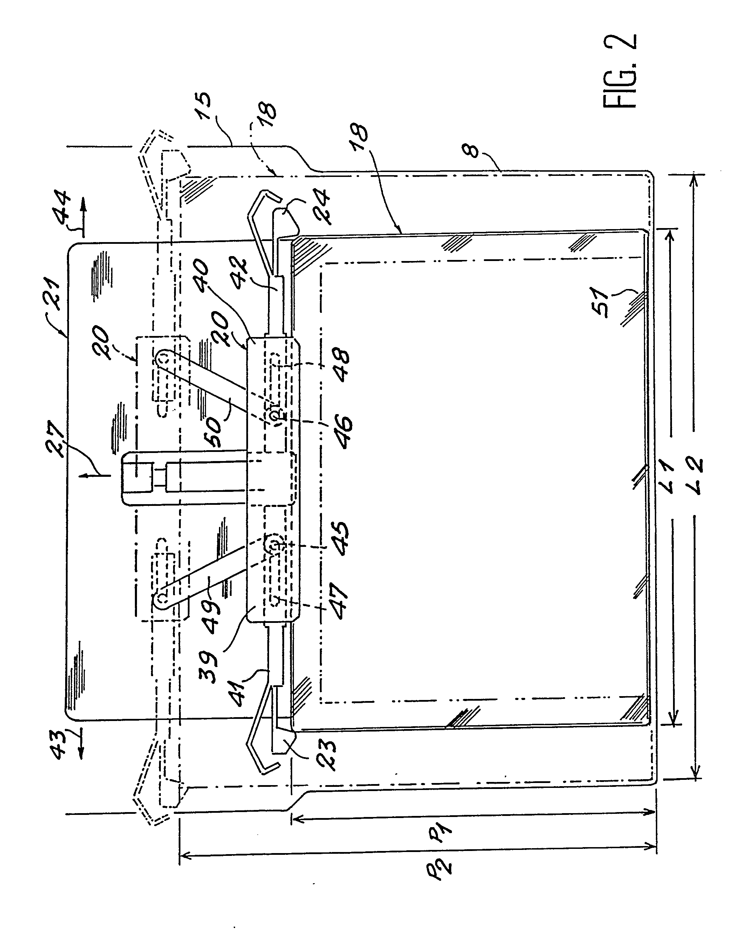

Grâce à ce montage, il est facile de comprendre en s'aidant de la figure 2, que le déplacement du curseur suivant la flèche 27 provoque le déplacement des bras dans le même sens de manière à passer de la largeur P1 à la largeur P2 de la cassette. Par ailleurs, les ergots 23 et 24 s'écartent l'un de l'autre de manière à passer de la longueur L1 à la longueur L2 de la cassette. On a ainsi réalisé un dispositif qui s'adapte à deux tailles de cassette L1 x P1 et L2 x P2.

Afin de bien maintenir en place la cassette 18 sur la platine 21, cette dernière comporte une butée 52 sur laquelle vient prendre appui le côté 51 de la cassette opposé à celui côté curseur, la cassette étant pressée contre cette butée 52 par un poussoir élastique 53 réalisé à l'intérieur du curseur. Par ailleurs, pour éviter l'échappement de la cassette vers le haut, la butée 52 et le curseur 20 sont prévus respectivement chacun avec un talon 54 et 55.

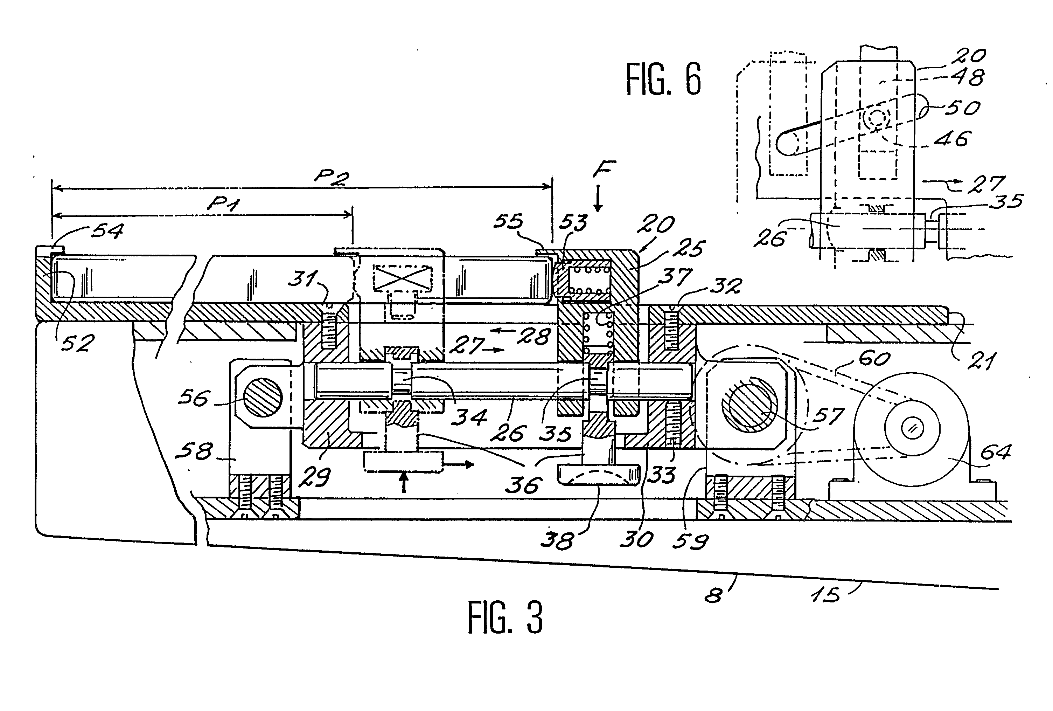

Afin de déplacer la cassette 18 dans le sens des flèches 43 ou 44, l'invention propose un mécanisme de déplacement de la platine 21. Ce mécanisme est réalisé par les pattes 29 et 30 (figure 3) qui coopèrent chacune avec un guide lisse 56 pour la patte 29 et un guide fileté 57 pour la patte 30, chaque guide étant fixé à la table 8 par un support 58 ou 59 et des vis 65 et 66. La patte 29 coulisse sur le guide lisse 56 par l'intermédiaire d'un étrier 62 tandis que la patte 30 comporte un alésage fileté ou noix d'entraînement 61 qui coopère avec le guide fileté 57. Le guide 56 est fixe tandis que le guide 57 tourne dans des paliers du support 59. La rotation du guide 57 est obtenue par une poulie 63, solidaire du guide 57, qui est par exemple entraînée par un moteur 64 par l'intermédiaire d'une courroie 60. La rotation du guide fileté 57 déplace la noix 61 dans le sens souhaité et il en est de même de la platine 21 et de la cassette supportée par cette dernière.

La figure 5-a montre l'une des positions extrêmes que peut prendre la petite cassette de dimensions L1 x P1 grâce au mécanisme de déplacement de la platine 21 le déplacement étant effectué suivant la flèche 43.

La figure 5-b montre l'autre position extrême que peut prendre la petite cassette de dimensions L1 x P1 grâce au mécanisme de déplacement pour un déplacement suivant la flèche 44.

## Revendications

1. Porte-cassette pour supporter sur une table (8) une cassette (18) contenant au moins un film sensible, comprenant
- une platine (21) qui est montée mobile par rapport à la table (8) suivant une première direction grâce à un premier dispositif de déplacement,
- un curseur (20) qui est monté mobile par rapport à la platine (21) suivant une deuxième direction perpendiculaire à la première grâce à un deuxième dispositif de déplacement, et
- des moyens associés au curseur (20) pour maintenir la cassette (18) en position par rapport à la platine (21) et s'adapter aux dimensions de la cassette(18), caractérisé en ce que le premier dispositif de déplacement comprend au moins deux guides (56,57) sur lesquels coulissent des pattes (29,30) solidaires de la platine (21), l'un des guides (57) étant fileté et coopérant avec une noix d'entraînement (61) de manière que la rotation du guide fileté (57) déplace la platine (21) dans le sens souhaité tandis que le second guide (56) est lisse.

2. Porte-cassette selon la revendication 1, caractérisé en ce que le premier dispositif de déplacement comprend en outre un moteur (64) qui entraîne en rotation le guide fileté (57).

3. Porte-cassette selon la revendication 1 ou 2, caractérisé en ce que le deuxième dispositif de déplacement comprend au moins un guide (26) qui est solidaire de la platine (21) et disposé perpendiculairement aux guides du premier dispositif de déplacement et sur lequel coulisse le curseur (20) de manière à prendre au moins deux positions correspondant chacune à une gorge (35,36) de verrouillage qui est portée par ledit guide et qui coopère avec des moyens de verrouillage déverrouillage portés par le curseur (20).

4. Porte-cassette selon la revendication 1,2 ou 3, caractérisé en ce que les moyens de maintien de la cassette associés au curseur comprennent deux ergots (23,24) qui sont portés par des bras (41,42) portés par le curseur (20) et qui viennent saisir deux des quatre coins de la cassette (18), lesdits bras comportant chacun un taquet (45,46) qui coopère avec une première ouverture (47,48) pratiquée dans le curseur (20) et avec une deuxième ouverture (49,50) pratiquée dans la platine (21) de sorte que le déplacement du curseur entraîne la modification de l'écartement des ergots (23,24) pour s'adapter aux dimensions de la cassette (18).

FIG. 1

FIG. 5 a

FIG. 5 b

FIG. 2

EP 0 395 458 A1

FIG. 6

FIG. 3

# FIG. 4

EP 0 395 458 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 196 088 (COMPAGNIE GENERALE DE RADIOLOGIE)<br>* page 8, lignes 1-14; page 5, ligne 15 - page 6, ligne 19; figure 2 *<br>--- | 1-4 | G 03 B 42/02<br>A 61 B 6/00 |
| A | US-A-4 357 538 (R.P. HUNT et al.)<br>* colonne 8, ligne 67 - colonne 11, ligne 17; figures 5-10 *<br>--- | 1-4 | |
| A | FR-A-1 477 391 (COMPAGNIE GENERALE DE RADIOLOGIE)<br>* page 1, colonne de gauche, lignes 19-24; page 2, colonne de droite, lignes 20-52; figure 2 *<br>----- | 1-4 | |

DOMAINES TECHNIQUES
RECHERCHES (Int. Cl.5)

G 03 B 42/00
A 61 B 6/00

Le présent rapport a été établi pour toutes les revendications

| Lieu de la recherche | Date d'achèvement de la recherche | Examinateur |
|---|---|---|
| BERLIN | 05-07-1990 | WEIHS J.A. |

EPO FORM 1503 03.82 (P0402)